# EUROPEAN PATENT SPECIFICATION

(11) **EP 4 193 925 B1**
(45) Date of publication and mention of the grant of the patent: **18.11.2026**
(21) Application number: 22212022.2
(22) Date of filing: 07.12.2022
(51) Int. Cl.: A61B 5/343, A61B 5/00, A61B 5/367, G06T 7/00, G06T 7/30, G06T 19/00, G16H 30/40, A61B 5/06, A61B 5/287

(54) **VISUALIZATION OF EPICARDIAL AND ENDOCARDIAL ELECTROANATOMICAL MAPS**
VISUALISIERUNG VON EPIKARDIALEN UND ENDOKARDIALEN ELEKTROANATOMISCHEN KARTEN
VISUALISATION DE CARTES ÉLECTROANATOMIQUES ÉPICARDIQUES ET ENDOCARDIAQUES

(30) Priority: 08.12.2021 US 202117545387
(43) Date of publication of application: 14.06.2023
(73) Proprietor: Biosense Webster (Israel) Ltd, 2066717 Yokneam (IL)
(72) Inventor: ALTMANN, Andres Claudio, 2066717 Yokneam (IL); GOVARI, Assaf, 2066717 Yokneam (IL)
(74) Representative: Carpmaels & Ransford LLP

(56) References cited:
- EP-A2- 1 897 490
- JP-A- H10 323 335
- US-A1- 2021 050 091
- TUNG RODERICK ET AL: "Simultaneous Endocardial and Epicardial Delineation of 3D Reentrant Ventricular Tachycardia", JOURNAL OF THE AMERICAN COLLEGE OF CARDIOLOGY, ELSEVIER, AMSTERDAM, NL, vol. 75, no. 8, 24 February 2020 (2020-02-24), pages 884 - 897, XP086061436, ISSN: 0735-1097, [retrieved on 20200224], DOI: 10.1016/J.JACC.2019.12.044
- ZHANG YONGJIE ET AL: "An atlas-based geometry pipeline for cardiac Hermite model construction and diffusion tensor reorientation", MEDICAL IMAGE ANALYSIS, vol. 16, no. 6, 1 August 2012 (2012-08-01), GB, pages 1130 - 1141, XP055789294, ISSN: 1361-8415, DOI: 10.1016/j.media.2012.06.005

## Description

### FIELD OF THE DISCLOSURE

The present disclosure relates generally to cardiac mapping, and particularly to visualizing anatomical cardiac maps.

### BACKGROUND OF THE DISCLOSURE

Various graphical tools for assisting the analysis of a rendering of an organ were proposed in the literature. For example, Tung et al. describe high-resolution simultaneous endocardial and epicardial mapping, in a paper titled, "Simultaneous Endocardial and Epicardial Delineation of 3D Reentrant Ventricular Tachycardia," Journal of the American College of Cardiology Volume 75, Issue 8, March 3, 2020, Pages 884-897. The authors use therein a 3D perspective view of the endocardial and epicardial surfaces.

As another example, U.S. Patent Application Publication No. 2007/0003119 describes display and navigation methods for multiple computer-aided detection (CAD) detections. A medical image is displayed to a viewer, and a request is received to instantiate CAD-assisted viewing. A timewise presentation sequence for the CAD detections is automatically computed according to a predetermined sequencing criterion. For each CAD detection, an expanded presentation 2-dimensional window floating on a computer screen is displayed for its associated location in the medical image, the expanded presentation windows being displayed according to the timewise presentation sequence.

Tung et al in JACC Vol. 75, No. 8, 2020 found that simultaneous endocardial and epicardial mapping with high-resolution technology improves the ability to characterize the 3D nature of scar-related reentrant ventricular tachycardia. US2021/050091A1 describes a mixed electroanatomical map coloring tool having a draggable geodesic overlay. EP 1897490 A2 discloses an endocardial map being registered with an epicardial map.

### SUMMARY OF THE INVENTION

The invention is as defined in the independent claims. Certain methods are described with reference to the system and/or computer program of the present invention. Whilst no claim is directed to these methods *per se,* the system and computer program are capable of being used and are intended to be used to implement such methods.

### BRIEF DESCRIPTION OF THE DRAWINGS

The present disclosure will be more fully understood from the following detailed description of the examples thereof, taken together with the drawings in which:
Fig. 1 is a schematic, pictorial illustration of a system for electroanatomical (EA) mapping, in accordance with an example of the present disclosure;
Fig. 2 shows schematic, pictorial volume renderings of exploded electroanatomical (EA) maps of endocardial and epicardial surfaces displayed on a graphical user interface (GUI), in accordance with an example of the present disclosure; and
Fig. 3 is a flow chart that schematically illustrates a method for generating the exploded EA maps of Fig. 2 and a related side-by side view, in accordance with an example of the present disclosure.

### DETAILED DESCRIPTION OF EXAMPLES

### OVERVIEW

Catheter-based electroanatomical (EA) mapping techniques may produce various EA maps of an organ, such as a left atrium of a heart. In some cases, to interpret the EA maps, a physician needs to compare two different EA maps. For example, during or after a cardiac EA mapping of a cardiac chamber, a physician may want to view EA maps of both the epicardial and endocardial surfaces of the cardiac chamber. However, the analysis task is difficult because, when both EA maps are presented to the physician overlaid on one another, one map obscures the other.

Some examples of the present disclosure that are described hereinafter provide a processor that receives a first representation of an internal surface of at least a portion of a wall of an organ of a patient, and a second representation of an external surface of at least the portion of the wall of the organ. The processor registers each representation with the other and generates an exploded representation to show both surfaces.

To this end, the processor generates first and second clipped views of the first and second representations, respectively, and displaces the clipped views along a predefined direction. The displacement causes the first and second clipped views to not overlap one another.

In some examples, the received first and second representations are EA maps of epicardial and endocardial surfaces, respectively, of a same portion of a cardiac chamber. In an example, a processor generates one surface 3D reconstruction (usually representing the endocardial anatomy). The epicardial surface is colored according to the epicardial electrical information projected from the mapping catheter position at the endocardial surface. The internal side of the map is colored according to the endocardial electrical signals. The displaced clipping views for visualization prevents either EA map being obscured at a selected portion.

In another example, the processor uses a colored scale, with a draggable threshold value, to differentiate between endocardial and epicardial electrical properties in a same region.

In another example, the processor visualizes the endocardial and the respective epicardial surfaces by placing a virtual camera internally to the map to display the endocardial information.

In an implementation, the processor uses only the outer surface and displays epicardial information in one texture and the endocardial in another texture. In this way, the correlated activation and the non-correlated activation is immediately visualized.

Typically, the processor is programmed in software containing a particular algorithm that enables the processor to conduct each of the processor-related steps and functions outlined above.

The disclosed techniques may assist the physician in the interpretation of epicardial and endocardial EA maps. The disclosed technique may thus expedite and improve the quality of complicated diagnostic tasks, such as those required in diagnostic catheterizations.

### SYSTEM DESCRIPTION

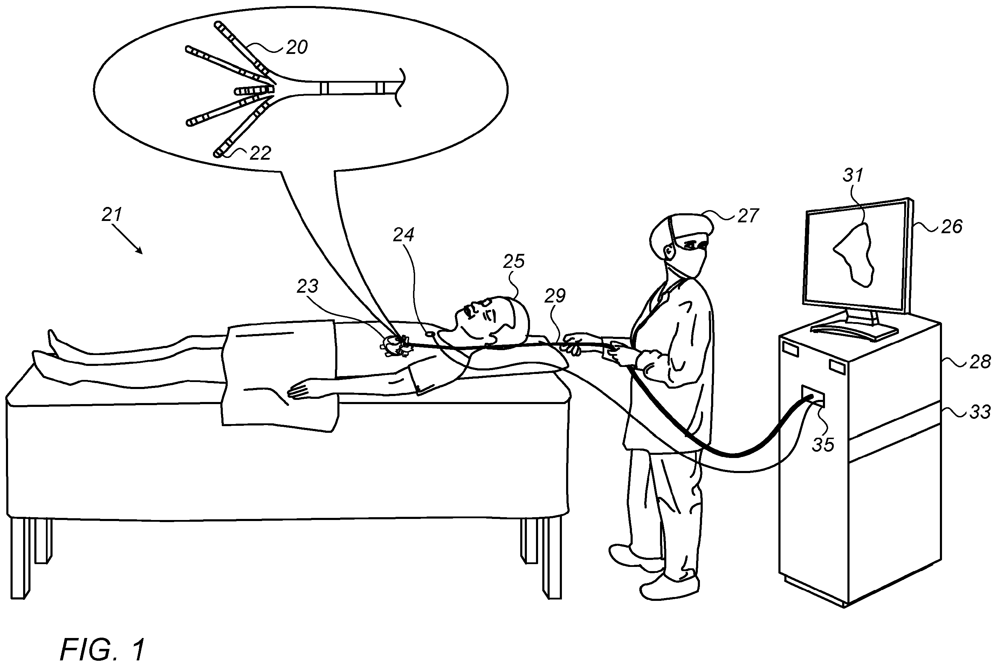

Fig. 1 is a schematic, pictorial illustration of a system 21 for electroanatomical (EA) mapping, in accordance with an example of the present disclosure. Fig. 1 depicts a physician 27 using an EA Pentaray^{®} catheter 29 to perform an EA mapping of a heart 23 of a patient 25. Catheter 29 comprises, at its distal end, one or more arms 20, which may be mechanically flexible, each of which is coupled with one or more electrodes 22. During the mapping procedure, electrodes 22 acquire and/or inject unipolar and/or bipolar signals from and/or to the tissue of heart 23. A processor 28 receives these signals via an electrical interface 35, and uses information contained in these signals to construct an exploded representation 31, such as exploded EA map 31 comprising EP values (e.g., LAT values), a color-coded endocardium, and epicardium surfaces that processor 28 stores in a memory 33. During and/or following the procedure, processor 28 may display EA map 31 on a display 26.

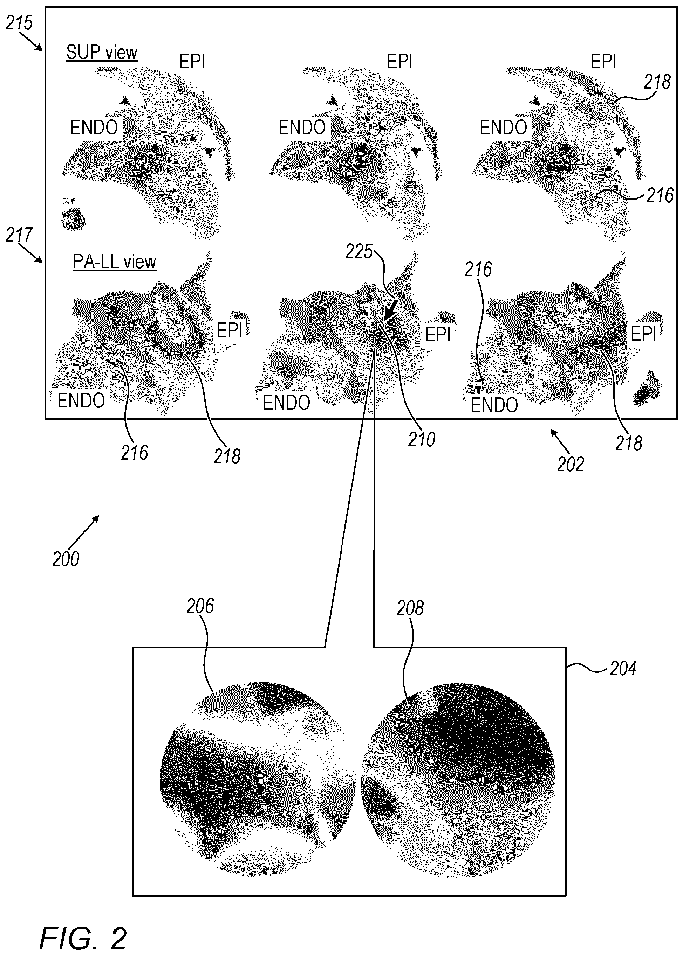

In some examples, exploded EA map 31 comprises a draggable circular overlay region, in order to display a side-by-side view of endocardium and epicardium surfaces of a same region of exploded EA map 31, as shown in Fig. 2.

During the procedure, a tracking system is used to track the respective locations of sensing-electrodes 22, such that each of the signals may be associated with the location at which the signal was acquired. For example, the Active Current Location (ACL) system, made by Biosense-Webster (Irvine California), which is described in US Patent 8,456,182 may be used. In the ACL system, a processor estimates the respective locations of the electrodes based on impedances measured between each of the sensing-electrodes 22, and a plurality of surface-electrodes 24, that are coupled to the skin of patient 25. For example, three surface-electrodes 24 may be coupled to the patient's chest, and another three surface electrodes may be coupled to the patient's back. For ease of illustration, only one surface-electrode is shown in Fig. 1. Electric currents are passed between electrodes 22 inside heart 23 of the patient, and surface electrodes 24. Processor 28 calculates an estimated location of all electrodes 22 within the patient's heart based on the ratios between the resulting current amplitudes measured at surface electrodes 24 (or between the impedances implied by these amplitudes) and the known positions of electrodes 24 on the patient's body. The processor may thus associate any given impedance signal received from electrodes 22 with the location at which the signal was acquired.

The example illustration shown in Fig. 1 is chosen purely for the sake of conceptual clarity. Other tracking methods can be used, such as ones based on measuring voltage signals. Other types of sensing catheters, such as the Lasso^{®} Catheter (produced by Biosense Webster) may equivalently be employed. Contact sensors may be fitted at the distal end of EA catheter 29. As noted above, other types of electrodes, such as those used for ablation, may be utilized in a similar way, fitted to electrodes 22 for acquiring the needed position data. Thus, an ablation electrode used for collecting position data is regarded, in this case, as a sensing electrode. In an optional example, processor 28 is further configured to indicate the quality of physical contact between each of the electrodes 22 and an inner surface of the cardiac chamber during measurement.

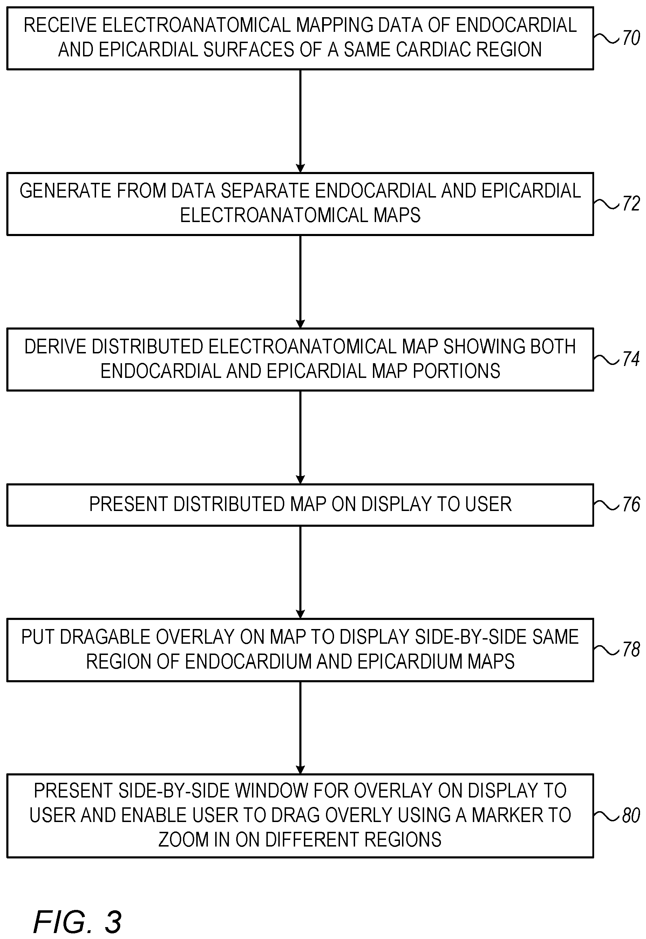

Processor 28 typically comprises a general-purpose computer with software programmed to carry out the functions described herein. In particular, processor 28 runs a dedicated algorithm as disclosed herein, including in Fig. 3, that enables processor 28 to perform the disclosed steps, as further described below. The software may be downloaded to the computer in electronic form, over a network, for example, or it may, alternatively or additionally, be provided and/or stored on non-transitory tangible media, such as magnetic, optical, or electronic memory.

### EPICARDIAL AND ENDOCARDIAL VIEW ON ONE 3D ELECTROANATOMICAL MAP

Fig. 2 shows schematic, pictorial volume renderings of exploded electroanatomical (EA) maps 202 of endocardial and epicardial surfaces displayed on a graphical user interface (GUI) 200, in accordance with an example of the present disclosure.

As seen, the processor has generated rendered clipped views of the endocardial and epicardial surfaces 216 and 218. Furthermore, the processor having displaced each of the clipped views along one predefined direction 215, and along a second predefined direction 217. Direction 215 is named in Fig. 2 "SUP view" and direction 217 is named therein "PA-LL view."

In another example, the processor receives first and second representations 216 and 218, in the form of the endocardial and epicardial surfaces 216 and 218, respectively.

As further seen, Fig. 2 shows a draggable circular overlay 210. A separate window 204 shows a side-by-side corresponding circular region color-coded EA map 206 of the endocardium surface and circular region color-coded EA map section 208 of the epicardium surface, for a same region defined by circular overlay 210. The physician may move circular overlay 210, for example, using a marker 225 to drag the circle, and also change the radius of circular overlay 210. Using overlay 210, the physician can, for example, quickly check whether or not a cardiac tissue region is entirely inactive without switching (i.e., toggling) between the two EA maps.

While Fig. 2 shows a circular overlay, the overly may have another shape, such as, for example, one that provides an isometric view that varies with location over the cardiac chamber. Furthermore, the disclosed technique may be used to create a multi-view map that comprises three or more overlay regions, with additional information shown on top of the endocardial and epicardial surface information, such as an overlay indicating, for example, cardiac wall thickness at the region.

Fig. 3 is a flow chart that schematically illustrates a non-claimed method for generating the exploded EA maps 202 and related side-by-side viewing window 204 of Fig. 2, in accordance with an example of the present disclosure.

The algorithm, according to the presented example, carries out a process that begins with processor 28 receiving EA data of endocardium and epicardium surfaces of a same region of a cardiac chamber, such as of left atrium 40, at EA data receiving step 70. In an example, the processor receives via interface circuits 35 EA mapping data acquired by catheter 29, as shown in Fig. 1. In another example, the processor uploads the EA data from memory 33 of system 21.

Next, at EA maps generation step 72, processor 28 generates separate endocardium and epicardium EA maps from the EA data. In alternative embodiment, the processor receives first and second surfaces that are, respectively, separate endocardium and epicardium EA maps.

Next, processor 28 derives an exploded EA map that shows both endocardium and epicardium EA map portions, at an exploded EA map derivation step 74. The processor may generate the exploded map by the method described in Fig. 2, e.g., providing views 202, or by other methods, such as using an exploded view.

At a map presentation step 76, processor 28 presents the exploded map on a display to a user, such as in views 202.

In an overlay generation step 78, processor 28 puts a draggable overlay 210 on the exploded EA map, to display on a window 204 side-by-side view of clipped views (216, 218) of a same region of the endocardium and epicardium EA maps.

Finally, at step 80, in response to a user moving marker 225, processor 28 drags side-by-side window 204 on view 202 to allow the user to zoom in on different regions of view 202, as shown in window 204.

The example flow chart shown in Fig. 3 is chosen purely for the sake of conceptual clarity. In optional examples, various additional steps may be performed, for example to automatically register additional layers, such as of medical images, and to generate and display respective overlay regions of the additional layers.

Although the examples described herein mainly address cardiac applications, the methods and systems described herein can also be used in other applications, such as in electroanatomical mapping of a brain.

It will thus be appreciated that the examples described above are cited by way of example, and that the present disclosure is not limited to what has been particularly shown and described hereinabove. Rather, the scope of the present invention, which is defined by the independent claims, may include both combinations and subcombinations of the various features described hereinabove, as well as variations and modifications thereof which would occur to persons skilled in the art upon reading the foregoing description.

## Claims

1. A system (21), comprising:
a memory (33) configured to store representations of surfaces of a wall of an organ of a patient; and
a processor (28), which is configured to:
receive a first representation of an internal surface of at least a portion of the wall of the organ of the patient, and a second representation of an external surface of at least the portion of the wall of the organ;
register the first and second representations with one another;
generate from the first and second representations an exploded representation (202) that shows both the internal surface and the external surface;
present the exploded representation to a user;
present a draggable overlay (210) on the exploded representation, wherein the overlay is configured to define a region of the exploded representation and the region is selected in response to positioning of the overlay by the user; and
display a view (206) of a region of the internal surface corresponding to the region defined by the overlay side-by-side with a view (208) of a region of the external surface corresponding to the region defined by the overlay.

2. The system (21) according to claim 1, wherein the processor is configured to generate the exploded representation (202) by generating first and second clipped views (216, 218) of the respective first and second representations, and displacing the first and second clipped views (216, 218) from one another along a predefined direction.

3. The system (21) according to claim 2, wherein the processor (28) is configured to displace the first and second clipped views (216, 218) from one another by causing the first and second clipped views to not overlap one another.

4. The system (21) according to claim 1, wherein the internal surface is an endocardium and the external surface is an epicardium.

5. The system (21) according to claim 4, wherein the first and second representations are electroanatomical (EA) maps.

6. The system (21) according to claim 5, wherein the EA maps comprise color coded EA maps.

7. The system (21) according to claim 5, wherein the EA maps are local activation time (LAT) maps.

8. The system (21) according to claim 1, wherein the overlay (210) has a circular shape.

9. A computer program comprising instructions that, when the program is executed by a processor, cause the processor to:
receive a first representation of an internal surface of at least a portion of a wall of an organ of a patient, and a second representation of an external surface of at least the portion of the wall of the organ;
register the first and second representations with one another;
generate from the first and second representations an exploded representation (202) that shows both the internal surface and the external surface;
present the exploded representation to a user;
present a draggable overlay (210) on the exploded representation, wherein the overlay is configured to define a region of the exploded representation and the region is selected in response to positioning of the overlay by the user; and
display a view of a region of the internal surface corresponding to the region defined by the overlay side-by-side with a view of a region of the external surface corresponding to the region defined by the overlay.

## Patentansprüche

1. System (21), umfassend:
einen Speicher (33), der konfiguriert ist, um Darstellungen von Oberflächen einer Wand eines Organs eines Patienten zu speichern; und
einen Prozessor (28), der konfiguriert ist zum:
Empfangen einer ersten Darstellung einer Innenoberfläche mindestens eines Abschnitts der Wand des Organs des Patienten und einer zweiten Darstellung einer Außenoberfläche mindestens des Abschnitts der Wand des Organs;
Registrieren der ersten und der zweiten Darstellung miteinander;
Erzeugen einer Explosionsdarstellung (202) aus der ersten und zweiten Darstellung, die sowohl die Innenoberfläche als auch die Außenoberfläche zeigt;
Präsentieren der Explosionsdarstellung einem Benutzer;
Präsentieren einer ziehbaren Überlagerung (210) auf der Explosionsdarstellung, wobei die Überlagerung konfiguriert ist, um einen Bereich der Explosionsdarstellung zu definieren und der Bereich als Reaktion auf ein Positionieren der Überlagerung durch den Benutzer ausgewählt wird; und
Anzeigen einer Ansicht (206) eines Bereichs der Innenoberfläche, der dem Bereich, der durch die Überlagerung definiert wird, entspricht, nebeneinander mit einer Ansicht (208) eines Bereichs der Außenoberfläche, der dem Bereich, der durch die Überlagerung definiert wird, entspricht.

2. System (21) nach Anspruch 1, wobei der Prozessor konfiguriert ist, um die Explosionsdarstellung (202) durch Erzeugen einer ersten und einer zweiten beschnittenen Ansicht (216, 218) der jeweiligen ersten und zweiten Darstellung und Verschieben der ersten und der zweiten beschnittenen Ansicht (216, 218) voneinander entlang einer vordefinierten Richtung zu erzeugen.

3. System (21) nach Anspruch 2, wobei der Prozessor (28) konfiguriert ist, um die erste und die zweite beschnittene Ansicht (216, 218) durch Veranlassen, dass die erste und die zweite beschnittene Ansicht einander nicht überlappen, voneinander zu verschieben.

4. System (21) nach Anspruch 1, wobei die Innenoberfläche ein Endokard ist und die Außenoberfläche ein Epikard ist.

5. System (21) nach Anspruch 4, wobei die erste und die zweite Darstellung elektroanatomische (EA) Karten sind.

6. System (21) nach Anspruch 5, wobei die EA-Karten farbcodierte EA-Karten umfassen.

7. System (21) nach Anspruch 5, wobei die EA-Karten Karten lokaler Aktivierungszeit (LAT) sind.

8. System (21) nach Anspruch 1, wobei die Überlagerung (210) eine runde Form aufweist.

9. Computerprogramm, umfassend Anweisungen, die, wenn das Programm durch einen Prozessor ausgeführt wird, den Prozessor veranlassen zum:
Empfangen einer ersten Darstellung einer Innenoberfläche mindestens eines Abschnitts einer Wand eines Organs eines Patienten und einer zweiten Darstellung einer Außenoberfläche mindestens des Abschnitts der Wand des Organs;
Registrieren der ersten und der zweiten Darstellung miteinander;
Erzeugen einer Explosionsdarstellung (202) aus der ersten und zweiten Darstellung, die sowohl die Innenoberfläche als auch die Außenoberfläche zeigt;
Präsentieren der Explosionsdarstellung einem Benutzer;
Präsentieren einer ziehbaren Überlagerung (210) auf der Explosionsdarstellung, wobei die Überlagerung konfiguriert ist, um einen Bereich der Explosionsdarstellung zu definieren und der Bereich als Reaktion auf das Positionieren der Überlagerung durch den Benutzer ausgewählt wird; und
Anzeigen einer Ansicht eines Bereichs der Innenoberfläche, der dem Bereich, der durch die Überlagerung definiert wird, entspricht, nebeneinander mit einer Ansicht eines Bereichs der Außenoberfläche, der dem Bereich, der durch die Überlagerung definiert wird, entspricht.

## Revendications

1. Système (21), comprenant :
une mémoire (33) configurée pour stocker des représentations de surfaces d'une paroi d'un organe d'un patient : et
un processeur (28) qui est configuré pour :
recevoir une première représentation d'une surface interne d'au moins une partie de la paroi de l'organe du patient, et une seconde représentation d'une surface externe d'au moins la partie de la paroi de l'organe ;
aligner les première et seconde représentations l'une avec l'autre ;
générer à partir des première et seconde représentations une représentation éclatée (202) qui montre à la fois la surface interne et la surface externe ;
présenter la représentation explosée à un utilisateur ;
présenter une superposition (210) déplaçable sur la représentation éclatée, dans lequel la superposition est configurée pour définir une région de la représentation éclatée et la région est sélectionnée en réponse au positionnement de la superposition par l'utilisateur ; et
afficher une vue (206) d'une région de la surface interne correspondant à la région définie par la superposition côte à côte avec une vue (208) d'une région de la surface externe correspondant à la région définie par la superposition.

2. Système (21) selon la revendication 1, dans lequel le processeur est configuré pour générer la représentation éclatée (202) en générant des première et seconde vues découpées (216, 218) des première et seconde représentations respectives, et en déplaçant les première et seconde vues découpées (216, 218) l'une par rapport à l'autre le long d'une direction prédéfinie.

3. Système (21) selon la revendication 2, dans lequel le processeur (28) est configuré pour déplacer les première et seconde vues découpées (216, 218) l'une de l'autre en amenant les première et seconde vues découpées à ne pas se chevaucher.

4. Système (21) selon la revendication 1, dans lequel la surface interne est un endocarde et la surface externe est un épicarde.

5. Système (21) selon la revendication 4, dans lequel les première et seconde représentations sont des cartes électroanatomiques (EA).

6. Système (21) selon la revendication 5, dans lequel les cartes EA comprennent des cartes EA codées par couleur.

7. Système (21) selon la revendication 5, dans lequel les cartes EA sont des cartes de temps d'activation locale (LAT).

8. Système (21) selon la revendication 1, dans lequel la superposition (210) a une forme circulaire.

9. Programme d'ordinateur comprenant des instructions qui, lorsque le programme est exécuté par un processeur, amènent le processeur à :
recevoir une première représentation d'une surface interne d'au moins une portion d'une paroi d'un organe d'un patient, et une seconde représentation d'une surface externe d'au moins la portion de la paroi de l'organe ;
aligner les première et seconde représentations l'une avec l'autre ;
générer à partir des première et seconde représentations une représentation éclatée (202) qui montre à la fois la surface interne et la surface externe ;
présenter la représentation explosée à un utilisateur ;
présenter une superposition (210) déplaçable sur la représentation éclatée, dans lequel la superposition est configurée pour définir une région de la représentation éclatée et la région est sélectionnée en réponse au positionnement de la superposition par l'utilisateur ; et
afficher une vue d'une région de la surface interne correspondant à la région définie par la superposition côte à côte avec une vue d'une région de la surface externe correspondant à la région définie par la superposition.
